# EUROPEAN PATENT APPLICATION

(11) **EP 4 527 394 A1**
(43) Date of publication of application: **26.03.2025**
(21) Application number: 23807586.5
(22) Date of filing: 15.05.2023
(51) Int. Cl.: A61K 35/14, A61K 9/08, A61K 35/19, A61K 47/02, A61K 47/12, A61M 1/02, A61P 7/08, A61P 17/02, A61P 19/02

(54) **NOVEL METHOD FOR PRESERVING PLATELETS**

(30) Priority: 16.05.2022 JP 2022080038
(71) Applicant: AdipoSeeds, Inc., Tokyo 151-0051 (JP)
(72) Inventor: URUGA, Yukako, Tokyo 151-0051 (JP); MATSUBARA, Yumiko, Tokyo 151-0051 (JP)
(74) Representative: Script Intellectual Property LLP
(86) International application number: PCT/JP2023/018026
(87) International publication number: WO 2023/223984

(57) **Abstract**

An object of the present invention is to provide a method for preserving a platelet in a refrigerated or frozen state while retaining more platelet functions, a platelet preservation solution for use in such a method, and the like. The present invention relates to a platelet preservation solution for refrigeration or freezing, comprising an electrolyte and an anticoagulant. The present invention also relates to a method for preserving a platelet, comprising: suspending a platelet in the platelet preservation solution according to the present invention; and preserving the suspension by refrigeration or freezing at 10°C or lower.

## Description

### Technical Field

The present invention relates to a novel method for preserving a platelet, a platelet preservation solution, and the like. More specifically, the present invention relates to a method for preserving a platelet in a refrigerated or frozen state while retaining more platelet functions, a platelet preservation solution for use in such a method, and the like.

### Background Art

Medical applications of platelets are mainly for blood transfusion and platelet-rich plasma (hereinafter, also referred to as "PRP") therapy. PRP is prepared by concentrating only platelets among three types of blood cells (red blood cells, white blood cells, and platelets) contained in blood to a high concentration, for example, through centrifugation of the blood of a subject. PRP contains, for example, cytokines such as epidermal growth factor (EGF), platelet-derived growth factor (PDGF), vascular endothelial growth factor (VEGF), insulin-like growth factor (IGF), fibroblast growth factor (FGF), hepatocyte growth factor (HGF), and transforming growth factor (TGF).

PRP is used in treatment of many diseases such as knee osteoarthritis and intractable skin ulcer (for example, Patent Document 1) and in cosmetic surgery. In PRP therapy, PRP is injected into an affected area. Platelets injected into the affected area produce cytokines in the affected area, and these cytokines stimulate regeneration of cells in the affected area and play an effective role in wound healing and tissue regeneration. Thus, it is extremely important to ensure that the cytokine-producing function of the platelets to be injected into the affected area is definitely retained in the PRP therapy. In PRP therapy, there has been a problem in that platelet functions decrease with time after blood collection (Patent Document 2).

For this reason, in the PRP therapy, it is common to prepare PRP from blood collected from a patient and then inject the PRP into the patient within a few hours, and when the PRP therapy is performed more than once, it is common to collect blood from a patient and prepare PRP each time it is performed. As described above, since in PRP therapy, it is extremely important to ensure that the cytokine-producing function of platelets to be injected into the affected area is definitely preserved, the possibility of preserving PRP for a few hours or more has not yet been contemplated in the first place, and neither has the possibility of preserving PRP by refrigeration or freezing.

On the other hand, a platelet formulation for blood transfusion is handled with a preservation period of about 4 days at room temperature from the date of manufacture. From the viewpoint of preserving platelet functions such as platelet aggregability, it needs to be preserved at room temperature.

### Prior Art Documents

### Patent Documents

Patent Document 1: Japanese unexamined Patent Application Publication No. 2009-195739
Patent Document 2: Japanese unexamined Patent Application Publication No. 2021-147333

### Summary of the Invention

### Object to be Solved by the Invention

An object of the present invention is to provide a method for preserving a platelet in a refrigerated or frozen state while retaining more platelet functions, a platelet preservation solution for use in such a method, and the like.

### Means to Solve the Object

As a result of intensive studies for solving the problem of the present invention, the present inventors have found, for example, that platelets can be preserved while retaining platelet functions by suspending the platelets in a platelet preservation solution containing an electrolyte and an anticoagulant and then preserving the suspension by refrigeration or freezing at 10°C or lower, thus completing the present invention.

That is, the present invention relates, for example, to:
(1) a platelet preservation solution for refrigeration or freezing, comprising an electrolyte and an anticoagulant;
(2) the platelet preservation solution according to (1), wherein the electrolyte is one or more selected from the group consisting of sodium chloride, potassium chloride, calcium chloride, magnesium chloride, sodium hydrogencarbonate, sodium lactate, and sodium acetate, and the anticoagulant is one or more selected from the group consisting of citric acid, ethylenediaminetetraacetic acid (EDTA), diethylenetriaminepentaacetic acid (DTPA), 1,2-diaminocyclohexanetetraacetic acid (DCTA), ethylene glycol bis(2-aminoethylether)tetraacetic acid (EGTA), oxalic acid, or salts thereof;
(3) a platelet-containing composition comprising the platelet preservation solution according to (1) or (2);
(4) a method for producing a platelet-containing composition, comprising suspending a platelet in the platelet preservation solution according to (1) or (2);
(5) a method for preserving a platelet, comprising: suspending a platelet in the platelet preservation solution according to (1) or (2); and preserving the suspension by refrigeration or freezing at 10°C or lower; and
(6) the method for preserving a platelet, according to (5), wherein suspending a platelet in the platelet preservation solution is suspending a platelet in a platelet preservation solution having a volume of 0.1 times or more with respect to the volume of plasma in the platelet.

### Effect of the Invention

According to the present invention, it is possible to provide a method for preserving a platelet in a refrigerated or frozen state while retaining more platelet functions, a platelet preservation solution for use in such a method, and the like.

### Brief Description of Drawings

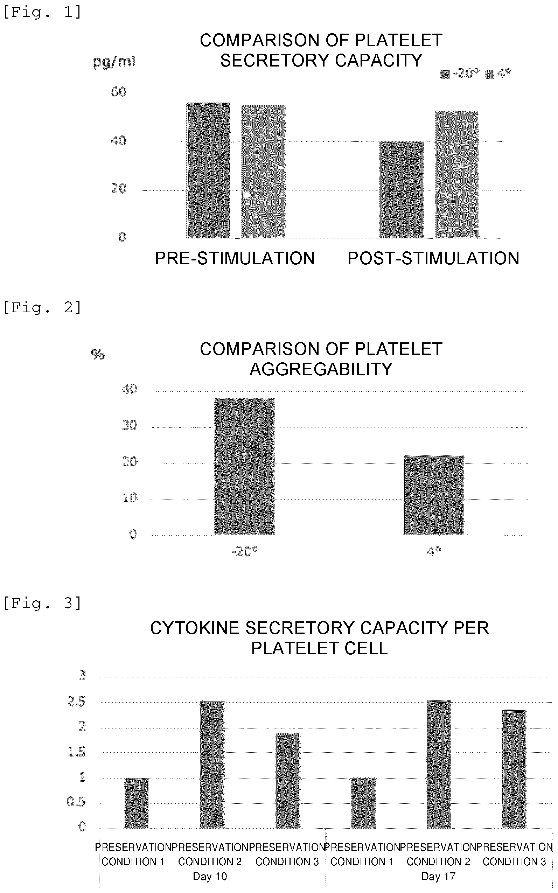

[Figure 1] Figure 1 is a graph showing results of comparing cytokine secretory capacity of platelets. The ordinate represents the concentration (pg/mL) of vascular endothelial growth factor (VEGF) in a culture medium of platelets. In both pre-stimulation and post-stimulation, the left bar represents results in the case of preservation at -20°C, and the right bar represents results in the case of preservation at 4°C.
[Figure 2] Figure 2 is a graph showing results of comparing aggregability of platelets. The ordinate represents the percent platelet aggregation (%). In the graph, the left bar represents results in the case of preservation at -20°C, and the right bar represents results in the case of preservation at 4°C. The results indicate retention of the adhesive function of membrane receptors of platelets and retention of the signaling ability of platelet aggregation mediated by membrane receptors.
[Figure 3] Figure 3 is a graph showing results of comparing cytokine secretory capacity per platelet cell fragment. The ordinate represents a ratio based on the concentration of VEGF in the case of stimulation with CaCl₂ after preservation under preservation condition 1.

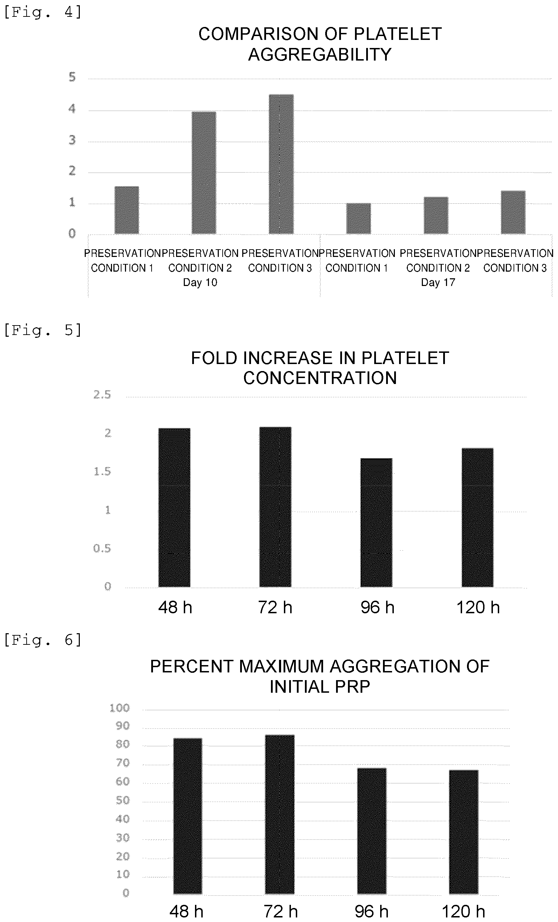

[Figure 4] Figure 4 is a graph showing results of comparing aggregability of platelets. The ordinate represents the percent platelet aggregation (%). The results indicate retention of the adhesive function of membrane receptors of platelets and retention of the signaling ability of platelet aggregation mediated by membrane receptors.
[Figure 5] Figure 5 is a graph showing results of comparing the fold increases in the platelet concentration. The ordinate represents the relative platelet concentration of a sample after concentration when the platelet concentration of the sample before concentration is defined as 1. The time on the abscissa represents the storage time of the donor blood used for sample preparation.
[Figure 6] Figure 6 is a graph showing results of comparing aggregability of platelets. The ordinate represents the percent maximum aggregation (%) of platelets. The time on the abscissa represents the storage time of the donor blood used for sample preparation.

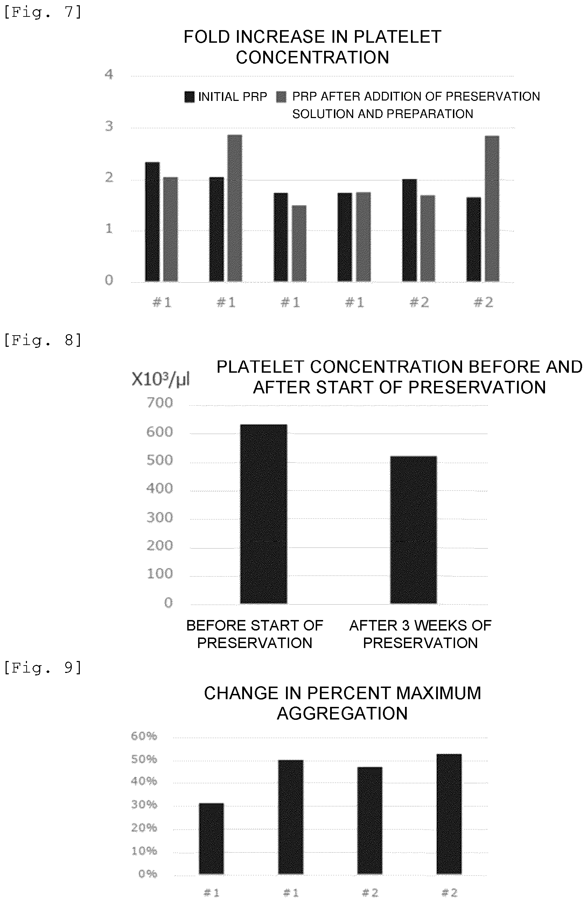

[Figure 7] Figure 7 is a graph showing results of comparing the fold increases in the platelet concentration. The ordinate represents the relative platelet concentration of a sample after concentration when the platelet concentration of the sample before concentration is defined as 1. #1 and #2 on the abscissa denote donor numbers assigned to identify each donor. Of the two bars arranged on the graph, the left bar represents the fold increase in the platelet concentration of the initial PRP, and the right bar represents the fold increase in the platelet concentration of PRP obtained by preserving the initial PRP in the platelet preservation solution of the present invention.
[Figure 8] Figure 8 is a graph showing results of comparing the platelet concentration before and after preservation with the platelet preservation solution of the present invention. The ordinate represents platelet concentration (× 10³ platelets/µL). The left bar represents the platelet concentration of the initial PRP before 3 weeks of preservation, and the right bar represents the platelet concentration of the initial PRP after 3 weeks of preservation.
[Figure 9] Figure 9 shows the ratio ("change in percent maximum aggregation") (%) of the maximum aggregability of platelets after preservation to the maximum aggregability of platelets before preservation. The ordinate represents the relative platelet concentration of a sample after concentration when the platelet concentration of the sample before concentration is defined as 1. #1 and #2 on the abscissa denote donor numbers assigned to identify each donor.

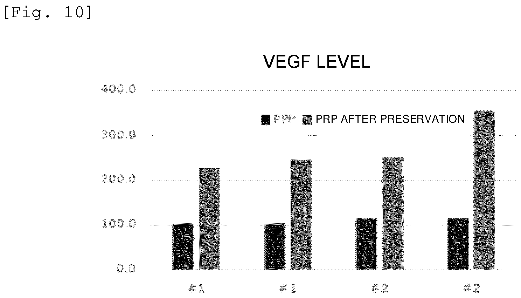

[Figure 10] Figure 10 is a graph showing results of comparing VEGF secretory capacity of platelets. The VEGF secretory capacity on the ordinate is expressed as a relative value when the VEGF secretory capacity of PRP prepared from blood donor #1 before preservation is defined as 100. #1 and #2 on the abscissa denote donor numbers assigned to identify each donor. Of the two bars arranged on the graph, the left bar represents the VEGF secretory capacity of PRP before 3 weeks of preservation, and the right bar represents the VEGF secretory capacity of PRP after 3 weeks of preservation.

### Mode of Carrying Out the Invention

The present invention includes embodiments such as:
[1] a platelet preservation solution for refrigeration or freezing (hereinafter, also referred to as the "platelet preservation solution of the present invention"), comprising an electrolyte and an anticoagulant;
[2] a platelet-containing composition (hereinafter, also referred to as the "platelet-containing composition of the present invention"), comprising the platelet preservation solution of the present invention and a platelet;
[3] a method for producing a platelet-containing composition (hereinafter, also referred to as the "production method of the present invention"), comprising suspending a platelet in the platelet preservation solution of the present invention; and
[4] a method for preserving a platelet (hereinafter, also referred to as the "preservation method of the present invention"), comprising:
   suspending a platelet in the platelet preservation solution of the present invention, and
   preserving the suspension by refrigeration or freezing at 10°C or lower.

### (Platelet)

The "platelet" in the present specification includes a mammalian platelet, more specifically, a platelet of, for example, a human, a mouse, a rat, a hamster, a guinea pig, a rabbit, a dog, a cat, a horse, a cow, a sheep, a pig, a goat, and a monkey, of which a human platelet is preferred.

The platelet in the present specification may be a platelet for blood transfusion but is preferably a platelet for promoting tissue repair, such as a platelet for PRP therapy, from the viewpoint of enjoying more of the significance of the present invention.

A method of platelet preparation is not particularly limited, and a platelet may be obtained by being isolated or concentrated from mammalian blood, or for example, obtained by inducing differentiation of a mammalian pluripotent stem cell and the like. Examples of the platelet obtained by being isolated or concentrated from mammalian blood include platelet-rich plasma (PRP) and a washed platelet, and from the viewpoint of ease of preparation, platelet-rich plasma is preferred. In addition, the platelet may not be a platelet derived from a subject to be administered, but from the viewpoint of a lower risk of rejection, is preferably a platelet derived from the subject to be administered.

The method of preparing a platelet from the blood of a mammal such as a human is not particularly limited, and any known method can be used. Specific examples thereof include a method of centrifuging the blood to obtain platelet-rich plasma. Examples of the centrifugation treatment include a method of performing centrifugation once (one step centrifugation) and a method of performing centrifugation twice (two step centrifugation). Examples of the one step centrifugation include a method of centrifugation at 100 to 300 G for 5 to 20 minutes, and examples of the two step centrifugation include a method of centrifugation at 100 to 300 G for 5 to 20 minutes followed by centrifugation at 200 to 300 G for 5 to 20 minutes. Note that when a platelet is prepared from blood, the blood may have been collected from a mammal and then stored. Examples of the storage period include 120 hours or less, 96 hours or less, 72 hours or less, and 48 hours or less. The storage temperature is not particularly limited as long as it is 10°C or lower regardless of whether the blood is preserved by refrigeration or freezing, but from the viewpoint of retaining more platelet functions, it is preferably higher than 0°C and 5°C or lower, or 0°C or lower, Examples of temperatures of 0°C or lower include -80°C or higher and 0°C or lower, -60°C or higher and -5°C or lower, -40°C or higher and -10°C or lower, and -30°C or higher and -15°C or lower.

### (Platelet Preservation Solution of the Invention)

The platelet preservation solution of the present invention is not particularly limited as long as it contains an electrolyte and an anticoagulant.

### (Electrolyte)

The "electrolyte" above is not particularly limited as long as an aqueous solution containing the electrolyte can be administered to any mammal for use, and examples thereof include one or more selected from the group consisting of sodium chloride, potassium chloride, calcium chloride, magnesium chloride, sodium hydrogencarbonate, sodium lactate, and sodium acetate.

The concentration of the electrolyte contained in the platelet preservation solution of the present invention is not particularly limited as long as the effects of the present invention are achieved, but is, for example, 160 to 400 mEq/L, preferably 180 to 360 mEq/L, and more preferably 180 to 320 mEq/L in terms of milliequivalent (mEq/L) of the total electrolyte.

In the preparation of the platelet preservation solution of the present invention, a powdered electrolyte may be used, or a commercially available electrolyte infusion solution or the like may be used. Examples of such an "electrolyte infusion solution" include physiological saline, Ringer's solution, Ringer's lactate solution (including sugar added Ringer's lactate solution), Ringer's acetate solution (including sugar added Ringer's acetate solution), and Ringer's bicarbonate solution (including those with sugar), of which Ringer's bicarbonate solution is preferred. These solutions may be used singly or in combinations of two or more thereof.

### (Anticoagulant)

Examples of the "anticoagulant" above include a chelating agent that prevents blood coagulation by coordinating calcium ions, which cause blood coagulation, and an antithrombin agent containing heparin that suppresses thrombin activity, which causes blood coagulation, among which a chelating agent is preferred. Examples of the anticoagulant as a chelating agent include one or more selected from the group consisting of citric acid, ethylenediaminetetraacetic acid (EDTA), diethylenetriaminepentaacetic acid (DTPA), 1,2-diaminocyclohexanetetraacetic acid (DCTA), ethylene glycol bis(2-aminoethylether)tetraacetic acid (EGTA), oxalic acid, or salts thereof; of which citric acid or a salt thereof is preferred. The salt is preferably an alkali metal salt, and more preferably a sodium salt.

Specific examples of an aqueous solution containing the anticoagulant (anticoagulant solution) include ACD-A solution (an aqueous solution containing sodium citrate hydrate, citric acid hydrate, and dextrose), CPD solution (an aqueous solution containing sodium citrate hydrate, citric acid hydrate, dextrose, and sodium dihydrogen phosphate hydrate), and CPDA-1 solution (an aqueous solution containing sodium citrate hydrate, citric acid hydrate, dextrose, sodium dihydrogen phosphate hydrate, and adenine), of which ACD-A solution is preferred.

The anticoagulants may be used singly or in combinations of two or more thereof. A commercially available anticoagulant and anticoagulant solution can be used.

The concentration of the anticoagulant in the preservation solution is not particularly limited as long as the effects of the present invention are achieved, and for example, when the anticoagulant is citric acid or a salt thereof, the concentration in terms of citric acid in the platelet preservation solution of the present invention is, for example, 0.2 to 3% w/v, preferably 0.5 to 2% w/v. Similarly, when the anticoagulant is EDTA or a salt thereof, the concentration in terms of EDTA is, for example, 0.2 to 3% w/v, preferably 0.5 to 2% w/v; when the anticoagulant is DTPA or a salt thereof, the concentration in terms of DTPA is, for example, 0.2 to 3% w/v, and preferably 0.5 to 2% w/v; when the anticoagulant is DCTA or a salt thereof, the concentration in terms of DCTA is, for example, 0.2 to 3% w/v, and preferably 0.5 to 2% w/v; when the anticoagulant is EGTA or a salt thereof, the concentration in terms of EGTA is, for example, 0.2 to 3% w/v, and preferably 0.5 to 2% w/v; and when the anticoagulant is oxalic acid or a salt thereof, the concentration in terms of oxalic acid is, for example, 0.2 to 3% w/v, and preferably 0.5 to 2% w/v. In addition, when two or more anticoagulants are used in combination, the total concentration thereof may be, for example, 0.2 to 3% w/v, and preferably 0.5 to 2% w/v.

In the preparation of the platelet preservation solution of the present invention, a powdered anticoagulant may be used, or a commercially available anticoagulant solution (an aqueous solution containing an anticoagulant) or the like may be used.

The method of preparing the platelet preservation solution of the present invention is not particularly limited, and the platelet preservation solution may be prepared by mixing an electrolyte, an anticoagulant, and water, or may be prepared by mixing an electrolyte infusion solution with an anticoagulant solution. In the case of using an electrolyte infusion solution and an anticoagulant solution, the volume ratio of the electrolyte infusion solution to the anticoagulant solution (electrolyte infusion solution: anticoagulant solution) is, for example, 0.5:1 to 10:1, preferably 1:1 to 5:1, more preferably 1.5:1 to 3.5:1, and even more preferably 1.8:1 to 3:1. In the case of using an electrolyte infusion solution and an anticoagulant solution, the concentration of the electrolyte infusion solution in the preservation solution is, for example, 20 to 90% v/v, preferably 40 to 90% v/v, more preferably 60 to 80% v/v, and even more preferably 65 to 75% v/v.

The osmotic pressure of the platelet preservation solution of the present invention is not particularly limited as long as the effects of the present invention are achieved, but is, for example, 230 to 400 mOsm/L, preferably 250 to 380 mOsm/L, more preferably 265 to 350 mOsm/L, even more preferably 270 to 330 mOsm/L, still more preferably 275 to 310 mOsm/L, and further preferably 280 to 300 mOsm/L.

### (Optional Ingredient)

The platelet preservation solution of the present invention may contain only an electrolyte, an anticoagulant, and water, or may contain other optional ingredients. Examples of such an optional ingredient include sugar such as glucose and maltose.

The concentration of the sugar contained in the platelet preservation solution of the present invention is not particularly limited as long as the effects of the present invention are achieved, but examples of the total concentration of the sugar in the platelet preservation solution include 0.1 to 25% w/v, 0.5 to 20% w/v, 1 to 10% w/v, and 2 to 5% w/v.

### (Container)

The platelet preservation solution of the present invention may be contained in a container. Examples of such a container include a bag, a syringe, an ampoule, and a vial.

### (Method for Preserving Platelet)

The method for preserving a platelet of the present invention is not particularly limited as long as it comprising:
suspending a platelet in the platelet preservation solution of the present invention; and
preserving the suspension by refrigeration or freezing at 10°C or lower.

The above "suspending a platelet in the platelet preservation solution of the present invention" (hereinafter also referred to as "suspension step") is not particularly limited as long as it is a step of bringing the platelet into a suspended state in the platelet preservation solution of the present invention. For example, a platelet may be added to and suspended in a platelet preservation solution; a platelet preservation solution may be added to a platelet for suspension; an electrolyte infusion solution may be added to a platelet, followed by addition of an anticoagulant to suspend the platelet; a platelet may be added to an electrolyte infusion solution, followed by addition of an anticoagulant to suspend the platelet; an electrolyte infusion solution and an anticoagulant may be added to a platelet for suspension; an anticoagulant may be added to a platelet, followed by addition of an electrolyte infusion solution to suspend the platelet; or a platelet may be added to an anticoagulant, followed by addition of an electrolyte infusion solution to suspend the platelet.

The amount of the platelet preservation solution of the present invention to be used in the suspension step is not particularly limited as long as the effects of the present invention are achieved, and for example, the platelet preservation solution has a volume of 0.1 times or more, preferably 0.3 times or more, more preferably 0.5 times or more, even more preferably 0.8 times or more, and still more preferably 1 time or more with respect to the volume of plasma in platelets, from the viewpoint of retaining more platelet functions. The upper limit of the amount of the platelet preservation solution of the present invention to be used is not particularly limited, and examples thereof include 1000 times or less, 500 times or less, and 200 times or less with respect to the volume of plasma in platelets.

The above " preserving the suspension by refrigeration or freezing at 10°C or lower" (hereinafter, also referred to as the "preservation step") is not particularly limited as long as it is a step of preserving the suspension obtained in the suspension step by refrigeration or freezing.

The preservation temperature in the preservation step is not particularly limited as long as it is 10°C or lower regardless of whether the blood is preserved by refrigeration or freezing, but from the viewpoint of retaining more platelet functions, it is preferably higher than 0°C and 5°C or lower, or 0°C or lower. Examples of temperatures of 0°C or lower include -80°C or higher and 0°C or lower, -60°C or higher and -5°C or lower, -40°C or higher and -10°C or lower, and -30°C or higher and -15°C or lower.

The preservation period of the preservation step is not particularly limited but is, for example, 3 hours or more, preferably 1 day or more, more preferably 3 days or more, even more preferably 7 days or more, and still more preferably 10 days or more, 15 days or more, or 25 days or more, from the viewpoint of enjoying more of the significance of the present invention. The upper limit of the preservation period is, for example, 6 months or less, 3 months or less, 2 months or less, or 1 month or less.

### (Method for Producing Platelet-Containing Composition)

The method for producing a platelet-containing composition of the present invention is not particularly limited as long as it includes a step of suspending a platelet (preferably PRP) in the platelet preservation solution of the present invention. The suspension step is as described above in the method for preserving a platelet of the present invention.

The method for producing a platelet-containing composition of the present invention preferably further includes a step of preparing a platelet (preferably PRP) before the suspending step.

Examples of the method of preparing a platelet include a method of isolating or concentrating a platelet from mammalian blood and a method of inducing differentiation of a mammalian pluripotent stem cell and the like into a platelet as described above, and from the viewpoint of simplicity, the method of isolating or concentrating a platelet from mammalian blood is preferred. Examples of the method of isolating or concentrating a platelet from mammalian blood include a method of centrifuging the blood, Examples of the centrifugation treatment include a method of performing centrifugation once (one step centrifugation) and a method of performing centrifugation twice (two step centrifugation). Examples of the one step centrifugation include a method of centrifugation at 100 to 300 G for 5 to 20 minutes, and examples of the two step centrifugation include a method of centrifugation at 100 to 300 G for 5 to 20 minutes followed by centrifugation at 200 to 300 G for 5 to 20 minutes.

### (Platelet-Containing Composition of the Invention)

The platelet-containing composition of the present invention is not particularly limited as long as it contains the platelet preservation solution of the present invention and a platelet. The platelet-containing composition of the present invention may be a platelet formulation.

The concentration of platelets in the platelet-containing composition of the present invention is not particularly limited, and is, for example, 1 × 10³ to 1 × 10³⁰ cells/mL, preferably 1 × 10⁵ to 1 × 10²⁵ cells/mL, and more preferably 1 × 10⁷ to 1 × 10²⁰ cells/mL.

The platelet-containing composition of the present invention may contain only the platelet preservation solution of the present invention and platelets or may contain other optional ingredients.

The platelet-containing composition of the present invention can be produced by mixing platelets with the platelet preservation solution of the present invention.

### (Retaining More Platelet Functions)

As used herein, the expression "retaining more platelet functions" means that more platelet functions are retained when platelets are preserved using the platelet preservation solution of the present invention than when the same platelets are preserved under the same preservation conditions without the platelet preservation solution of the present invention. In addition, as used herein, the "platelet functions" means cytokine secretory capacity and/or platelet aggregability. A preferred example of the cytokine secretory capacity is VEGF secretory capacity.

Hereinafter, the present invention will be described in detail with reference to Examples, but the present invention is not limited to Examples.

### Examples

### [Test 1] Evaluation of Platelet Preservation Temperature

The following test was performed to determine whether platelet functions was retained when the platelets were preserved by refrigeration or freezing.

### (1. Preparation of PRP)

Blood was collected from blood donors. The blood was centrifuged at 200 G for 10 minutes (one step centrifugation) or done at 200 G for 10 minutes followed by centrifugation at 200 to 300 G for 10 minutes (two step centrifugation) to obtain PRP.

### (2. Preservation of PRP)

PRP was placed in a syringe, sealed, and then preserved under a condition of 4 °C or -20°C for one month.

### (3. Evaluation of Platelet Functions in PRP)

### (1) Evaluation of Cytokine Secretory Capacity

To the PRP after preservation were added CaCl₂ at 4.5 mM, adenosine diphosphate (ADP) at 5 µM, and collagen at 2.5 µg/mL to perform platelet stimulation. The concentration of vascular endothelial growth factor (VEGF) in the culture medium was measured before the start of platelet stimulation and within 1 hour after the start of platelet stimulation. The results are shown in Figure 1.

The results in Figure 1 indicated that the cytokine secretory capacity was retained even after one month of preservation at either a temperature of 4°C or -20°C. It was also shown that the cytokine secretory capacity was better retained in the case of preservation at 4°C than in the case of preservation at -20°C (Figure 1).

### (2) Evaluation of Platelet Aggregability

To the PRP after preservation were added CaCl₂ at 4.5 mM, ADP at 5 µM, and collagen at 2.5 µg/mL as platelet aggregation inducers, and the percent platelet aggregation (%) was determined using a platelet aggregability measuring device (PRP313M, manufactured by TAIYO Instruments, Inc.). The results are shown in Figure 2.

The results in Figure 2 indicated that the platelet aggregability was retained even after one month of preservation at either a temperature of 4°C or -20°C. It was also shown that the platelet aggregability was better retained in the case of preservation at -20°C than in the case of preservation at 4°C (Figure 2).

### [Test 2] Evaluation 1 of Influence of Platelet Preservation Solution of the Invention

The following test was performed to determine how the platelet preservation solution of the present invention affected the retention of platelet functions.

### (1. Preparation of PRP)

Blood was collected from blood donors. The blood was centrifuged at 200 G for 10 minutes (one step centrifugation) or done at 200 G for 10 minutes followed by centrifugation at 200 to 300 G for 10 minutes (two step centrifugation) to obtain PRP.

### (2. Preparation of Platelet Preservation Solution of the Invention)

Ringer's bicarbonate solution ("BICANATE" manufactured by Otsuka Pharmaceutical Factory, Inc. or "BICARBON" manufactured by AY Pharmaceuticals Co., Ltd.) and ACD-A solution (manufactured by Terumo Corporation) were provided and mixed so that the volume ratio of the Ringer's bicarbonate solution to the ACD-A solution was 2.3:1 to prepare the platelet preservation solution of the present invention.

The composition of the Ringer's bicarbonate solution ("BICARBON") is as shown in Table 1, and the composition of the ACD-A solution is as shown in Table 2.

**[Table 1]**

| Ringer's bicarbonate solution | | |
|---|---|---|
| Ingredient Name | in 500 mL | Concentration |
| Sodium chloride | 3.07 g | 0.614 w/v% |
| Potassium chloride | 0.15 g | 0.03 w/v% |
| Calcium chloride hydrate | 0.11 g | 0.022 w/v% |
| Magnesium chloride | 0.051 g | 0.0102 w/v% |
| Sodium hydrogencarbonate | 1.05 g | 0.21 w/v% |
| Sodium citrate hydrate | 0.245 g | 0.049 w/v% |

**[Table 2]**

| ACD-A solution | |
|---|---|
| Ingredient Name | Concentration |
| Sodium citrate hydrate | 2.20 w/v% |
| Citric acid hydrate | 0.80 w/v% |
| Dextrose | 2.20 w/v% |

Note that the ACD-A solution contained 2.2% w/v sodium citrate hydrate (C₆H₅Na₃O₇·2H₂O), 0.8% w/v citric acid hydrate (C₆H₈O₇·H₂O), and the Ringer's bicarbonate solution contained 0.049% w/v sodium citrate hydrate (C₆H₅Na₃O₇·2H₂O). Therefore, the citric acid concentration (concentration in terms of citric acid) in the platelet preservation solution of the present invention, obtained by mixing the Ringer's bicarbonate solution and the ACD-A solution at a volume ratio of 2.3:1, was about 0.75% w/v. The electrolyte in the Ringer's bicarbonate solution was 286 mEq/L, and the electrolyte in the ACD-A solution was 57 mEq/L. Therefore, the electrolyte in the platelet preservation solution of the present invention, obtained by mixing the Ringer's bicarbonate solution and the ACD-A solution at a volume ratio of 2.3:1, was approximately 217 mEq/L.

### (3. Preservation of PRP)

Three preservation conditions were set for PRP.

### Preservation condition 1 (PRP only)

PRP was placed in a syringe, sealed, and then preserved under a condition of 4°C or -20°C for 10 days or 17 days.

### Preservation condition 2 (PRP: 50% and Platelet Preservation Solution: 50%)

PRP and the platelet preservation solution of the present invention were placed in a syringe and sealed so that the volume ratio of plasma in the PRP to the platelet preservation solution of the present invention was 50:50, and then preserved under a condition of 4°C or -20°C for 10 days or 17 days.

### Preservation condition 3 (PRP: 65% and Platelet Preservation Solution: 35%)

PRP and the platelet preservation solution of the present invention were placed in a syringe and sealed so that the volume ratio of plasma in the PRP to the platelet preservation solution of the present invention was 65:35, and then preserved under a condition of 4°C for 10 days or 17 days.

### (4. Evaluation of Platelet Functions in PRP)

### (1) Evaluation of Cytokine Secretory Capacity

The VEGF secretory capacity of each PRP after preservation was evaluated in the same manner as described in Test 1 above. Note that the cytokine secretory capacity (VEGF secretory capacity) was evaluated per platelet cell fragment. The cytokine secretory capacity (VEGF secretory capacity) was expressed as a ratio based on the concentration of VEGF in the case of stimulation with CaCl₂ after the PRP was preserved under preservation condition 1. The results are shown in Figure 3.

From Figure 3, it was shown that about 2 to 2.5 times more cytokine secretory capacity was retained in both cases of preservation conditions 2 and 3 using the platelet preservation solution of the present invention than in the case of preservation condition 1 without the platelet preservation solution of the present invention.

### (2) Evaluation of Platelet Aggregability

The platelet aggregability of each PRP after preservation was evaluated in the same manner as described in Test 1 above. Note that the platelet aggregability was expressed as a ratio based on the percent platelet aggregation (%) after preservation under preservation condition 1. The results are shown in Figure 4.

From Figure 4, it was shown that platelet aggregability was better retained in both cases of preservation conditions 2 and 3 using the platelet preservation solution of the present invention than in the case of preservation condition 1 without the platelet preservation solution of the present invention. It was also shown that the platelet aggregability was better retained in the case of preservation at 4°C for 10 days than in the case of preservation at 4°C for 17 days.

### [Test 3] Influence of Long-Term Storage of Blood Sample

In Test 1 and Test 2, PRP prepared from blood immediately after collection from a donor was used. The following test was then performed in the case of using PRP prepared from blood collected from a donor and stored for a long time.

### (1. Preparation of Initial PRP)

Blood was collected from blood donors. This blood was stored under a condition of 4°C for a predetermined period of time (48 hours, 72 hours, 96 hours, or 120 hours). Thereafter, each blood was centrifuged at 100 to 300 G for 10 minutes (one step centrifugation) or done at 100 to 300 G for 10 minutes followed by centrifugation at 200 to 2500 G for 10 to 20 minutes (two step centrifugation) to obtain initial PRP.

### (2. Preparation of Platelet Preservation Solution of the Invention)

The platelet preservation solution of the present invention was produced by the method described in Test 2 above.

### (3. Evaluation of the Fold Increase in the Platelet Concentration)

Figure 5 shows the results of evaluating the fold increase in the platelet concentration of the initial PRP. As can be seen from Figure 5, even in the case where the storage time of the blood (whole blood) of the donor was 120 hours, the fold increase in the platelet concentration of the initial PRP was retained at approximately the same level as in the case where the storage time was 48 hours or the like.

### (4. Evaluation of Platelet Aggregability)

The platelet aggregability of the initial PRP was evaluated in the same manner as described in Test 1 above. The results are shown in Figure 6. As can be seen from Figure 6, even in the case where the storage time of the blood (whole blood) of the donor was 120 hours, the percent maximum aggregation of the initial PRP was retained at approximately the same level as in the case where the storage time was 48 hours or the like.

### [Test 4] Evaluation 2 of Influence of Platelet Preservation Solution of the Invention

The following test was performed in the case of using PRP prepared from blood collected from a donor and stored for a long time to determine how the platelet preservation solution of the present invention affected retention of platelet functions in the PRP.

### (1. Preparation of Initial PRP)

Blood was collected from blood donors. This blood was stored under a condition of 4°C for 120 hours. Thereafter, each blood was centrifuged at 100 to 300 G for 10 minutes (one step centrifugation) or done at 100 to 300 G for 10 minutes followed by centrifugation at 200 to 2500 G for 10 to 20 minutes (two step centrifugation) to obtain initial PRP.

### (2. Preparation of Platelet Preservation Solution of the Invention)

The platelet preservation solution of the present invention was produced by the method described in Test 2 above.

### (3. Preservation of PRP)

The initial PRP and the platelet preservation solution of the present invention were placed in a syringe and sealed so that the volume ratio of plasma in the PRP to the platelet preservation solution of the present invention was 20:7, and then preserved under a condition of 4°C for 3 weeks.

### (4. Evaluation of the Fold Increase in the Platelet Concentration)

Figure 7 shows the results of evaluating the fold increase in the platelet concentration of the initial PRP and the fold increase in the platelet concentration of the initial PRP preserved in the platelet preservation solution of the present invention for 3 weeks. As can be seen from Figure 7, in all samples, the fold increase in the platelet concentration of the PRP preserved in the platelet preservation solution of the present invention was retained at approximately the same level as that of the initial PRP.

### (5. Evaluation of Platelet Concentration)

Figure 8 shows the results of measuring the platelet concentration before and after the initial PRP was preserved for 3 weeks in the section of (3. Preservation of PRP) above. As can be seen from Figure 8, when PRP was preserved in the platelet preservation solution of the present invention, the platelet concentration was retained at a sufficiently high level even after long-term preservation for as long as 3 weeks.

### (6. Evaluation of Platelet Aggregability)

In the section of (3. Preservation of PRP) above, the platelet aggregability before and after the initial PRP was preserved for 3 weeks was evaluated in the same manner as described in Test 1 above. Figure 9 shows the ratio of the maximum aggregability of platelets after preservation to the maximum aggregability of platelets before preservation (i.e., the "change in percent maximum aggregation"). As can be seen from Figure 9, when PRP was preserved in the platelet preservation solution of the present invention, the percent maximum aggregation was relatively sufficiently retained even after long-term preservation for as long as 3 weeks.

Note that when the platelet aggregability of the initial PRP that had been preserved for 3 weeks under a condition of 4°C without addition of the platelet preservation solution of the present invention was evaluated, no aggregability was observed.

### (7. Evaluation of VEGF Secretory Capacity)

In the section of (3. Preservation of PRP) above, the VEGF secretory capacity before the initial PRP was preserved for 3 weeks and the VEGF secretory capacity of PRP after preservation for 3 weeks were evaluated in the same manner as described in Test 2 above. The VEGF secretory capacity was expressed as a relative value when the VEGF secretory capacity of PRP prepared from blood donor #1 before preservation was defined as 100. The results are shown in Figure 10. The results in Figure 10 indicated that when PRP was preserved in the platelet preservation solution of the present invention, the VEGF secretory capacity was twice or more as high as that before preservation.

Note that when the VEGF secretory capacity of the initial PRP that had been preserved for 3 weeks under a condition of 4°C without addition of the platelet preservation solution of the present invention was evaluated, no secretion of VEGF was observed.

### Industrial Applicability

According to the present invention, it is possible to provide a method for preserving a platelet in a refrigerated or frozen state while retaining more platelet functions, a platelet preservation solution for use in such a method, and the like.

## Claims

1. A platelet preservation solution for refrigeration or freezing, comprising an electrolyte and an anticoagulant.

2. The platelet preservation solution according to claim 1, wherein the electrolyte is one or more selected from the group consisting of sodium chloride, potassium chloride, calcium chloride, magnesium chloride, sodium hydrogencarbonate, sodium lactate, and sodium acetate, and the anticoagulant is one or more selected from the group consisting of citric acid, ethylenediaminetetraacetic acid (EDTA), diethylenetriaminepentaacetic acid (DTPA), 1,2-diaminocyclohexanetetraacetic acid (DCTA), ethylene glycol bis(2-aminoethylether)tetraacetic acid (EGTA), oxalic acid, or salts thereof.

3. A platelet-containing composition comprising the platelet preservation solution according to claim 1 or 2 and a platelet.

4. A method for producing a platelet-containing composition, comprising suspending a platelet in the platelet preservation solution according to claim 1 or 2.

5. A method for preserving a platelet, comprising:
suspending a platelet in the platelet preservation solution according to claim 1 or 2; and
preserving the suspension by refrigeration or freezing at 10°C or lower.

6. The method for preserving a platelet according to claim 5, wherein suspending a platelet in the platelet preservation solution is suspending a platelet in a platelet preservation solution having a volume of 0.1 times or more with respect to the volume of plasma in the platelet.
